# FASCICULE DE BREVET EUROPEEN

(11) **EP 0 975 640 B1**
(45) Date de publication et mention de la délivrance du brevet: **30.06.2004**
(21) Numéro de dépôt: 98920607.3
(22) Date de dépôt: 10.04.1998
(51) Int. Cl.: C07D 519/04, A61K 31/395

(54) **DERIVES HALOGENES ANTIMITOTIQUES D'ALCALOIDES DE VINCA**
ANTIMITOTISCHE HALOGENIERTE DERIVATE VON VINCA ALKALOIDEN
VINCA ALKALOID ANTIMITOTIC HALOGENATED DERIVATIVES

(30) Priorité: 10.04.1997 FR 9704398
(43) Date de publication de la demande: 02.02.2000
(60) Demande divisionnaire: 03017052.6
(73) Titulaire: PIERRE FABRE MEDICAMENT, 92100 Boulogne (FR)
(72) Inventeur: DUFLOS, Alain, F-81290 Labruguière (FR); FAHY, Jacques, F-81290 Labruguière (FR); THILLAYE DU BOULAY, Valérie, F-81310 Lisle-sur-Tarn (FR); BARRET, Jean-Marc, F-81100 Castres (FR); HILL, Bridget, F-81100 Castres (FR)
(74) Mandataire: Ahner, Francis
(86) Numéro de dépôt international: PCT/FR1998/000730
(87) Numéro de publication internationale: WO 1998/045301

(56) Documents cités:
- FR-A- 2 707 988
- JACQUES FAHY ET AL: "Vinca alkaloids in superacidic media: a method for creating a new family of antitumor derivatives." JOURNAL OF THE AMERICAN CHEMICAL SOCIETY., vol. 119, no. 36, - 10 septembre 1997 pages 8576-8577, XP002072890 DC US

## Description

Les alcaloïdes dimères du *Catharanthus roseus* et leurs dérivés, couramment appelés alcaloïdes de Vinca, sont largement utilisés en chimiothérapie anticancéreuse depuis une trentaine d'années. Ils sont représentés par quatre produits :
- deux composés naturels, la vinblastine et la vincristine,
- deux produits d'hémisynthèse, la vindésine obtenue à partir de la vinblastine, et la vinorelbine, synthétisée à partir des alcaloïdes monomères précurseurs, catharanthine et vindoline.

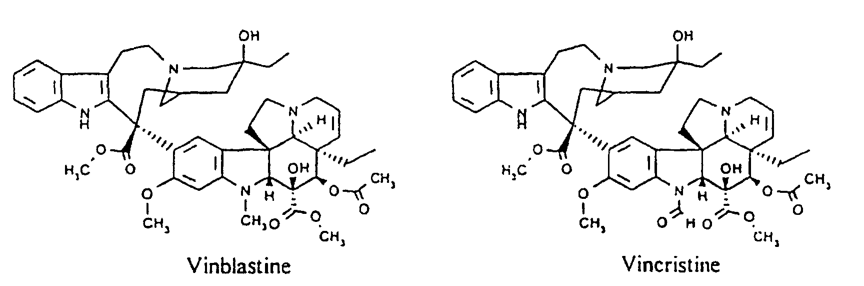

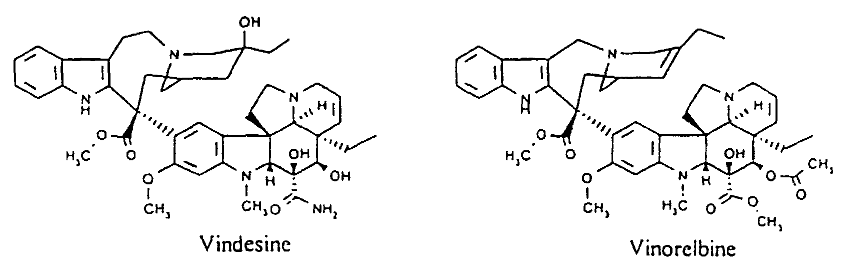

### Structure des alcaloïdes de Vinca utilisés en chimiothérapie

Dans le cadre de nos travaux de recherche visant à obtenir de nouveaux dérivés de cette famille pouvant conduire à de nouvelles applications en chimiothérapie anticancéreuse, nous avons adopté une démarche originale consistant à utiliser la réactivité exceptionnelle des milieux superacides, susceptible d'induire de profondes modifications dans ces molécules hautement fonctionnalisées.

Nous avons ainsi déjà préparé une série de composés difluorés en position 20', qui sont inaccessibles aujourd'hui par les méthodes classiques de synthèse (brevet FR 2 707 988 du 21.07.93, WO 95/03312), et dont les propriétés pharmacologiques sont particulièrement intéressantes.

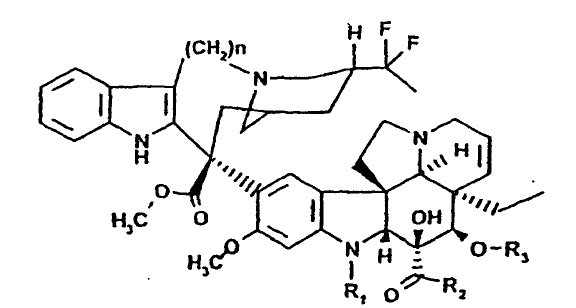

### Formule générale des composés décrits dans le brevet FR 2 707 988

L'application de cette chimie inhabituelle à ce type de molécules complexes nous a permis de préparer une nouvelle famille de composés halogénés, également inaccessibles par la chimie classique.

La présente invention, réalisée au Centre de Recherche Pierre Fabre, a pour objet des dérivés d'alcaloïdes de Vinca, leur mode de préparation et leur application en thérapeutique.

Les composés de l'invention possèdent la formule générale **1**, schématisée ci-dessous : 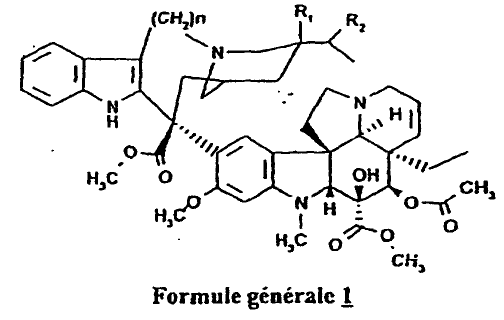 dans laquelle :
n= 1 ou 2,
R₁ représente un atome d'hydrogène ou de fluor,
R₂ représente un atome de chlore.

L'invention concerne également les sels des composés de formule générale **1** avec des acides minéraux ou organiques pharmaceutiquement acceptables. L'acide employé peut être, à titre d'exemple non limitatif, l'acide sulfurique ou l'acide tartrique.

L'invention concerne aussi bien les isomères correspondant aux configurations **R** et **S** des carbones 4' et 20' des composés de formule générale **1**, que leurs mélanges en toute proportion.

Les dérivés de l'invention sont préparés par réaction d'un composé de formule générale **2** en milieu superacide, provenant du mélange d'un acide fort de Bronsted comme l'acide fluorhydrique, et d'un acide de Lewis fort comme le pentafluorure d'antimoine, en présence d'un réactif générateur d'espèces de type superélectrophile, suivant la dénomination proposée par G. Olah (*Ang. Chem. Int. Ed. Engl.,* **32**, 767-88, 1993). Ce réactif peut être constitué d'un dérivé chloré comme le chlorure de méthylène, le chloroforme, le tétrachlorure de carbone, le 2,2-dichloropropane ou d'un mélange de ces dérivés en toutes proportions.

La structure des composés de formule **2** est décrite ci-dessous : 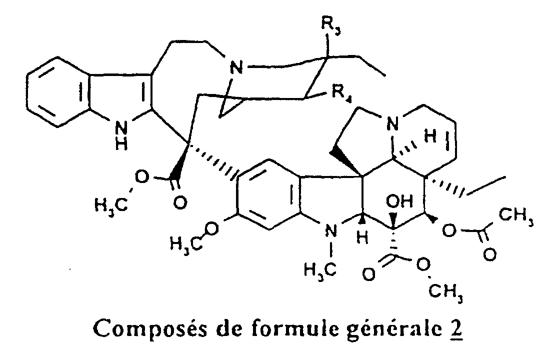 dans laquelle :
**R**_{**3**} représente un groupement hydroxyle et **R**_{**4**} un atome d'hydrogène, dans ce cas le composé **2** correspond à la vinblastine,
   ou bien :
**R**_{**3**} et **R**_{**4**} formant une double liaison, dans ce cas le composé de formule **2** correspond à la 3',4'-anhydrovinblastine.

Les réactions en milieu superacide s'effectuent dans des récipients resistant à l'acide fluorhydrique comme le Téflon® ou un acier de composition adaptée. Le dérivé de formule **2**, dans laquelle **R**_{**3**} et **R**_{**4**} sont définis comme ci-dessus, est dissous dans l'acide fluorhydrique ou dans un dérivé chloré défini comme ci-dessus servant de solvant, et ajouté au mélange superacide, qui peut éventuellement déjà contenir une fraction de dérivé chloré. Cette addition est effectuée en maintenant la température du milieu à une valeur choisie, comprise entre -80 et -30 °C.

A basse température et en présence de chlorure de méthylène ou de tétrachlorure de carbone, les composés de formule **1** dans laquelle **n** = 2, **R**_{**1**} = H et **R**_{**2**} = Cl sont isolés de façon majoritaire. Les composés de formule **1** dans laquelle **n** = 2, **R**_{**1**} = F et **R**_{**2**} = Cl sont isolés du même milieu réactionnel en proportion plus faible. Ceci est résumé dans le schéma ci-dessous :

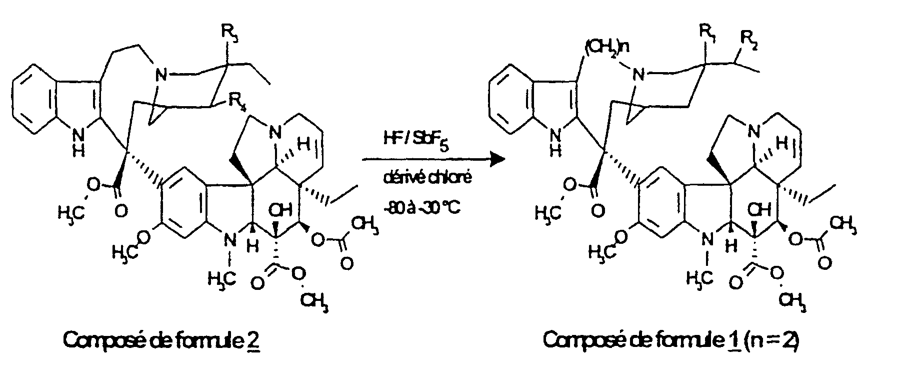

Les dérivés de formule générale **1**, dans lesquels **n** = 1, **R**_{**1**} et **R**_{**2**} étant définis comme ci-dessus, sont préparés par contraction du cycle C' (**n** = 2 → **n** = 1) des composés de Formule générale **1** dans lesquels **n** = 2, suivant les méthodes décrites dans la littérature (*Eur. J. Med. Chem*., **18**, 419-24, 1983), en particulier par action de la N-bromosuccinimide dans le chlorure de méthylène en présence d'un acide comme l'acide trifluoroacétique et à température inférieure à -10 °C. Après neutralisation en milieu basique, la 9'-bromoindolénine du dérivé de formule **1** dans laquelle **n** = 2, intermédiaire peu stable et non isolé, subit une hydrolyse de préférence dans un mélange [chlorure de méthylène : eau : tétrahydrofuranne], en présence ou non de tétrafluoroborate d'argent (AgBF₄), à une température comprise entre - 10° C et le reflux du solvant. L'addition d'AgBF₄; en quantité catalytique ou stoechiométrique par rapport au composé **1** ( **n** = 2), permet d'accélérer le cours de la réaction, si celle-ci n'est pas terminée après l'étape de neutralisation.

Le produit majoritaire de la réaction correspond au composé de formule **1** dans laquelle **n** = 1. Cette réaction est schématisée ci-dessous:

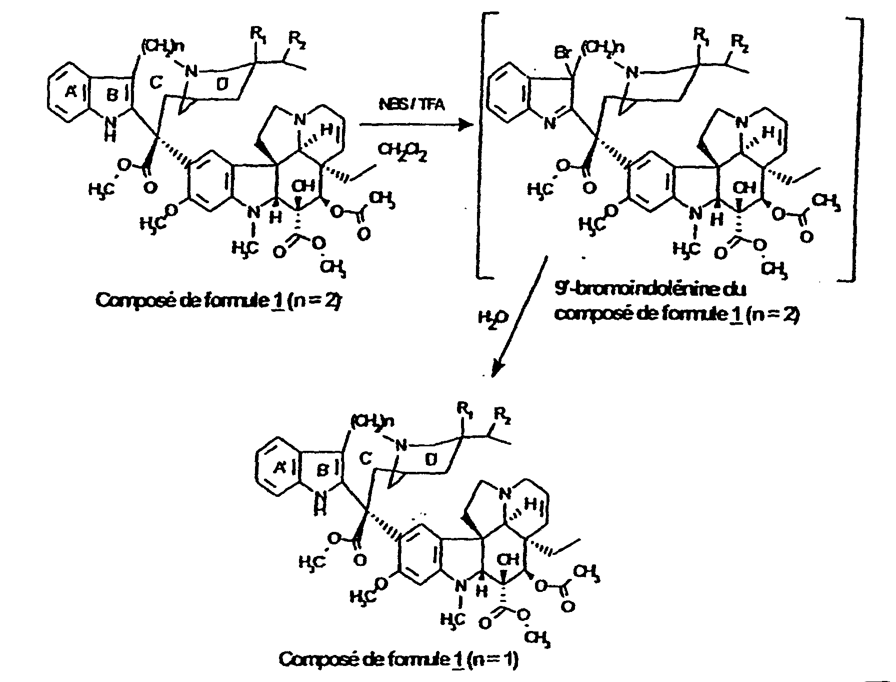

Les exemples suivants illustrent l'invention, sans toutefois en limiter la portée. Les caractéristiques spectroscopiques confirment la structure des composés obtenus selon l'invention.

### Exemple 1 :

20'-chloro-4'-déoxyvinblastine **1.**
(**n** = 2, **R**_{**1**} = H, **R**_{**2**} = Cl)

Dans un récipient en Téflon de 250 ml, on prépare une solution de pentafluorure d'antimoine (60 g; 0,28 mole) dans 80 ml (4 moles) d'acide fluorhydrique anhydre que l'on refroidit à -60 **°**C. Sous agitation magnétique, on y ajoute successivement 1,63 ml (17 mmoles) de tétrachlorure de carbone, puis goutte-à-goutte une solution de 13,75 g (17 mmoles) de 3',4'-anhydrovinblastine 2 (**n** = 2, **R**_{**3**} et **R**_{**4**} formant une double liaison) dans 25 ml de chlorure de méthylène, en évitant que la température ne dépasse -40 °C. Après 30 minutes, le milieu réactionnel est versé avec grande précaution dans 1,5 litre d'une suspension aqueuse 3 M de Na₂CO₃ contenant 200 ml de chlorure de méthylène. Après décantation, la phase aqueuse est extraite par 100 ml de chlorure de méthylène. Les phases organiques sont regroupées, lavées par une solution saturée de NaCl, séchées sur MgSO₄ et évaporées.

L'analyse par CLHP analytique du résidu récupéré montre la présence de deux pics voisins intégrant au total pour 41% (21 et 20%) notés produit de l'**Exemple 1a** et produit de l'**Exemple 1b** dans les tableaux décrivant les spectres de RMN, correspondant aux deux diastéréoisomères en 20' de la 20'-chloro-4'-déoxyvinblastine.

La purification est effectuée par chromatographie sur colonne de silice, puis par CLHP préparative en phase inverse.

Les deux diastéréoisomères de la 20'-chloro-4'-déoxyvinblastine séparés sont salifiés sous forme de ditartrate par addition de deux équivalents (mole) d'acide tartrique dans l'eau, et lyophilisés.
C₄₆ H₅₇ Cl N₄ O₈ , 2(C₄ H₆ O₆) : 1129,62
**Point de fusion :** > 260 °C

| | | | | | |
|---|---|---|---|---|---|
| **IR (KBr)** | 3448.17 | 1736.05 | 1615.36 | 1459.86 | 1372.51 |
| | 1231.89 | 1121.18 | 1067.54 cm⁻¹. | | |

**Spectre de masse (D/CI NH**_{**3**}**) :**
MH ⁺ = 829.4

### Exemple 2 :

20'-chloro-4'-déoxyvinblastine **1.**
(**n** = 2, **R**_{**1**} = H, **R**_{**2**} = Cl)

Ce composé est obtenu en suivant le mode opératoire décrit à l'**Exemple 1**, en remplaçant la 3',4'-anhydrovinblastine **2** (**n** = 2, **R**_{**3**} et **R**_{**4**} formant une double liaison) par la vinblastine **2** (**n** = 2, **R**_{**3**} = OH, **R**_{**4**} = H).

Les caractéristiques physico-chimiques et spectroscopiques des produits isolés sont identiques à celles des composés obtenus à l'**Exemple 1**.

### Exemple 3 :

20'-chloro-4'-déoxy-4'-fluorovinblastine **1.**
(**n** = 2, **R**_{**1**} = F, **R**_{**2**} = Cl)

Ce composé est isolé du mélange réactionnel obtenu à l'**Exemple 1** ou à l'**Exemple 2**, à partir d'autres fractions moins polaires provenant de la chromatographie sur colonne de silice. La purification finale est effectuée par CLHP préparative en phase inverse.

La 20'-chloro-4'-déoxy-4'-fluorovinblastine est salifiée sous forme de ditartrate par addition de deux équivalents (mole) d'acide tartrique dans l'eau, et lyophilisée.
C₄₆ H₅₆ Cl F N₄ O₈ , 2(C₄ H₆ O₆) : 1147,61
**Point de fusion :** > 260 °C

| | | | | | |
|---|---|---|---|---|---|
| **IR (KBr)** | 3470.13 | 2951.45 | 1735.92 | 1616.62 | 1458.44 |
| | 1371.64 | 1228.52 | 1040.10 | 743.07 cm⁻¹. | |

**Spectre de masse (D/CI NH**_{**3**}**) :**
MH ⁺ = 847.4

### Exemple 4 :

20'-chloro-3',4'-dihydrovinorelbine **1.**
(**n** = 1, **R**_{**1**} = H, **R**_{**2**} = Cl)

On dissout 900 mg (1,08 mmoles) de 20'-chloro-4'-déoxyvinblastine **1** (**n** = 2, **R**_{**1**} = H, **R**_{**2**} = Cl), isolée suivant l'**Exemple 1**, dans 5 ml de chlorure de méthylène, additionné de 95 µl (1,2 mmoles) d'acide trifluoroacétique, refroidi à -45 °C. On place le montage à l'abri de la lumière, et on ajoute en solution dans 2 ml de CH₂Cl₂ et 95 µl (1,2 mmoles) de TFA 193 mg (1,08 mmoles) de N-bromosuccinimide, et on laisse agiter pendant 30 minutes. Le mélange est alors neutralisé par addition de 5 ml d'une solution saturée de NaHCO₃, puis aussitôt d'une solution de 211 mg (1,08 mmoles) de tétrafluoroborate d'argent dans un mélange de 5 ml de tétrahydrofuranne et 2 ml d'eau. On laisse revenir à température ambiante sous agitation magnétique pendant environ deux heures.

Après filtration, la phase organique est séparée, lavée par deux fois 10 ml d'eau, puis 10 ml d'une solution de NaCl saturé. Après séchage sur MgSO₄, la solution est évaporée et le résidu, est purifié par chromatographie sur colonne de silice, puis par CLHP préparative en phase inverse.
C₄₅ H₅₅ Cl N₄ O₈ : 815,41

| | | | | | |
|---|---|---|---|---|---|
| **IR (KBr)** | 3446 | 2950 | 1740.89 | 1459.16 | 1246.59 |
| | 1200.65 | 1042 cm⁻¹. | | | |

**Spectre de masse (D/CI NH**_{**3**}**) :**
MH ⁺ = 815.4

### Exemple 5 :

20'-chloro-3',4'-dihydro-4'-fluorovinorelbine **1.**
(**n** = 1, **R**_{**1**} = F, **R**_{**2**} = Cl)

Ce composé est obtenu en suivant le mode opératoire décrit à l'**Exemple 4,** en remplaçant la 20'-chloro-4'-déoxyvinblastine par la 20'-chloro-4'-déoxy-4'-fluorovinblastine **1** (**n** = 2, **R**_{**1**} = F, **R**_{**2**} = Cl). La 20'-chloro-3',4'-dihydro-4'-fluorovinorelbine est purifiée par chromatographie dans des conditions identiques.
C₄₅ H₅₄ Cl F N₄ O₈ : 833,40

| | | | | | |
|---|---|---|---|---|---|
| **IR (KBr)** | 3446.83 | 2950.98 | 1742.73 | 1617.73 | 1457.36 |
| | 1234.38 | 1042.23 | 996.50 | 742.15 cm⁻¹. | |

**Spectre de masse (D/CI NH**_{**3**}**) :**
MH ⁺ = 833.5

Les déplacements chimiques des protons caractéristiques des structures nouvelles préparées ci-dessus sont rassemblés dans le tableau suivant :

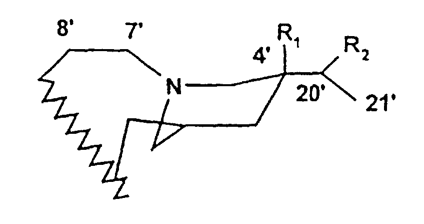

| Atome d'hydrogène | Produit de l'**Exemple** | | | |
|---|---|---|---|---|
| | **1a et 1b** | **3** | **4** | **5** |
| H_{4'} | 1,95 (m, 1H) | - | 1,90 (m, 1H) | - |
| H_{7'} | 3,1-3,3 (m, 2H) | 3,1 (m, 1H) | 4,32 (d, 1H) | 4,38 (d, 1H) |
| | | 3,3 (m, 1H) | 4,46 (d, 1H) | 5,07 (d, 1H) |
| H_{8'} | 3,1-3,25 (m,2H) | 3,05 (m, 1H) | - | - |
| | | 3,45 (m,1H) | | |
| H_{20'} | 3,68 (m, 1H) | 3,80 (m, 1H) | 3,80 (m, 1H) | 3,93 (m, 1H) |
| H_{21'} | 1,50 (d, 3H) | 1,44 (d,3H) | 1,50 (d, 3H) | 1,57 (d, 3H) |

La description des spectres de RMN ¹³C des produits nouveaux obtenus suivant les exemples ci-dessus est détaillée dans le tableau suivant :

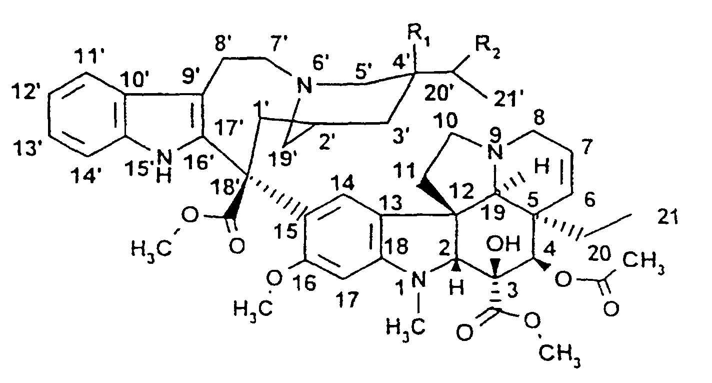

### Numérotation des alcaloïdes de Vinca (suivant les recommandations I.U.P.A.C.)

| Caractéristiques de RMN ¹³C des composés préparés | | | | | |
|---|---|---|---|---|---|
| **Exemple** | **1a** | **1b** | **3** | **4** | **5** |
| Carbone n° | | | | | |
| 1 | N | N | N | N | N |
| 2 | 83.348 | 83.348 | 82.72 | 83.5 | 83.26 |
| 3 | 79.71 | 79.71 | 79.01 | 80.07 | 79.74 |
| 4 | 76.496 | 76.466 | 75.78 | 76.78 | 76.46 |
| 5 | 42.69 | 42.69 | 42.04 | 42.95 | 42.66 |
| 6 | 130.04 | 130.01 | 129.41 | 130.31 | 130.08 |
| 7 | 124.583 | 124.583 | 123.89 | 124.94 | 124.54 |
| 8 | 50.27 | 50.209 | 49.64 | 50.75 | 50.49 |
| 9 | N | N | N | N | N |
| 10 | 50.27 | 50.209 | 49.64 | 50:55 | 50.38 |
| 11 | 44.6 | 44.63 | 44.03 | 44.8 | 44.48 |
| 12 | 53.332 | 53.332 | 52.63 | 53.55 | 53.23 |
| 13 | 122.673 | 122.733 | 122.07 | 123 | 122.64 |
| 14 | 123.431 | 123.461 | 122.87 | 123.37 | 123.17 |
| 15 | 121.066 | 121.036 | 120.48 | 120.2 | 120.84 |
| 16 | 158.086 | 158.056 | 157.52 | 158.3 | 158.12 |
| 17 | 94.142 | 94.142 | 93.63 | 94.19 | 94.01 |
| 18 | 152.75 | 152.719 | 152.07 | 153.01 | 152.65 |
| 19 | 65.369 | 65.399 | 64.83 | 65.64 | 65.45 |
| 20 | 30.835 | 30.835 | 30.17 | 31 | 30.71 |
| 21 | 8.368 | 8.399 | 7.72 | 8.42 | 8.1 |
| 22 | 38.415 | 38.385 | 37.76 | 38.65 | 38.45 |
| 23 | 171.669 | 171.669 | 171.06 | 171.95 | 171.69 |
| 24 | 52.271 | 52.271 | 51.57 | 52.51 | 52.23 |
| 25 | 55.849 | 55:849 | 55.29 | 56.04 | 55.49 |
| 26 | 170.941 | 170.941 | 170.13 | 171.22 | 170.88 |
| 27 | 21.193 | 21.193 | 20.45 | 21.45 | 21.15 |
| 1' | 33.594 | 33.594 | 33 | 32.24 | 33.59 |
| 2' | 30.016 | 30.077 | 28.56 | 29.67 | 29.71 |
| 3' | 34.867 | 35.929 | 35.4 | 34.85 | 35.4 |
| 4' | 38.779 | 39.355 | 92.52 | 31 | 95.92 |
| 5' | 56.819 | 57.243 | 58.43 | 55 | 56.7 |
| 6' | N | N | N | N | N |
| 7' | 56.819 | 56.788 | 55.4 | 47.38 | 46.98 |
| 8' | 28:986 | 29:076 | 27.69 | Absent | Absent |
| 9' | 117.064 | 117.185 | 116.07 | 110.76 | 111.68 |
| 10' | 129.252 | 129.282 | 128.72 | 129.09 | 128.7 |
| 11' | 118.337 | 118.398 | 117.7 | 118.77 | 118.23 |
| 12' | 118.883 | 118.913 | 118.2 | 120.1 | 119.56 |
| 13' | 122.339 | 122.37 | 121.63 | 123.04 | 122.46 |
| 14' | 110.575 | 110.545 | 109.9 | 110.76 | 110.52 |
| 15' | 135.073 | 135.073 | 134.45 | 134.98 | 134.69 |
| 16' | N | N | N | N | N |
| 17' | 130.617 | 130.495 | 130.49 | 133.3 | 133.43 |
| 18' | 55.454 | 55.424 | 54.95 | 55.04 | 55.77 |
| 19' | 47.42 | 47.45 | 46.11 | 48.6 | 47.04 |
| 20' | 62.155 | 62.428 | 60.7 | 62.7 | 61.24 |
| 21' | 22.406 | 22.649 | 18.5 | 23.05 | 18.64 |
| 22' | 174.883 | 174.883 | 174.17 | 175 | 175 |
| 23' | 52.45 | 52.453 | 51.7 | 52.96 | 52.62 |

Comme les alcaloïdes antitumoraux du *Catharanthus roseus,* les composés préparés suivant l'invention sont des "poisons du fuseau mitotique".

Cette propriété a été confirmée par la mesure de l'inhibition de la polymérisation de la tubuline en microtubules en présence des composés de l'invention, en suivant la méthode décrite par R. C. Weisenberg (*Science* **177**, 1196-7, 1972). Les résultats sont exprimés en IC₅₀, qui correspond à la concentration de composé qui provoque 50 % d'inhibition de la polymérisation. Ce phénomène est aisément suivi et quantifié par l'intermédiaire des variations de la densité optique.

A titre d'exemple, le tableau ci-dessous montre les résultats obtenus avec deux dérivés préparés suivant l'invention :

| **Produit** | **IC**_{**50**} **(µM)** |
|---|---|
| **Exemple 1** | 1.99 |
| **Exemple 3** | 1.54 |
| **Vinorelbine** | 1.70 |

Compte-tenu de cette propriété pharmacologique caractéristique des alcaloïdes de Vinca, les composés de la présente invention peuvent être utilisés en chimiothérapie anticancéreuse.

Les préparations pharmaceutiques contenant ces principes actifs peuvent être mises en forme pour l'administration par voie orale, intraveineuse ou sous-cutanée, de manière classique, bien connue de l'homme du métier.

## Revendications

1. Dérivés antimitotiques des alcaloïdes de Vinca correspondant à la formule générale suivante : 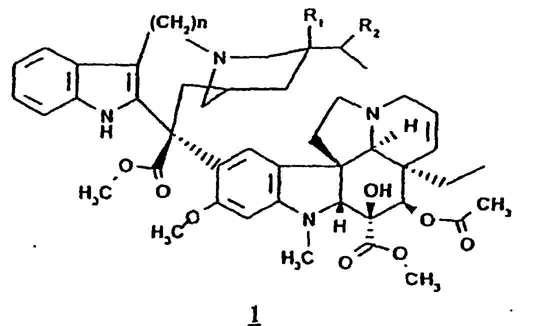 dans laquelle :
**n** = 1 ou 2,
**R**_{**1**} représente un atome d'hydrogène ou de fluor,
**R**_{**2**} représente un atome de chlore,
ainsi que leurs sels avec des acides minéraux ou organiques thérapeutiquement acceptables, et les mélanges des diastéréoisomères correspondant aux configurations des carbones 4' et 20' des composés et leurs mélanges en toute proportion.

2. Composés de formule générale **1** selon la revendication 1, **caractérisés par le fait qu'**ils sont choisis parmi : 20'-chloro-4'-deoxy-4'-fluorovinblastine, 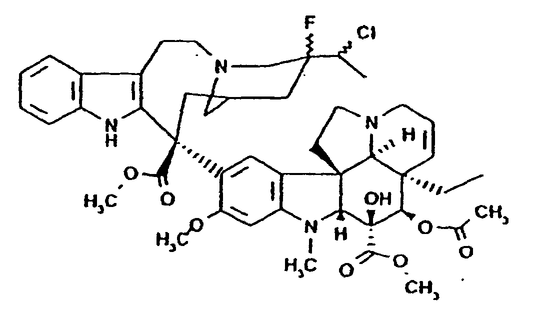 20'-chloro-4'-deoxyvinblastine: 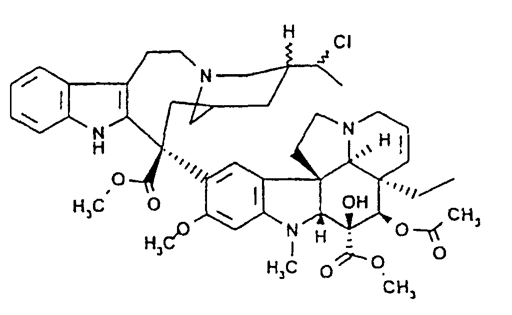 20'-chloro-3',4'-dihydrovinorelbine : 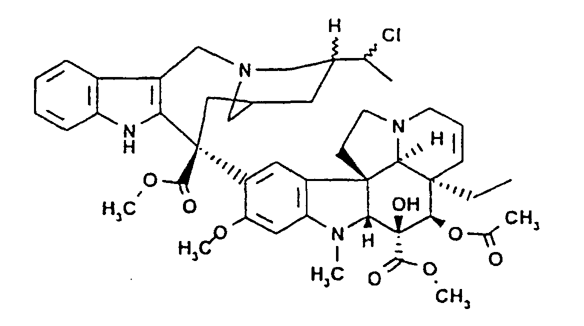 20'-chloro-3',4'-dihydro-4'-fluorovinorelbine :

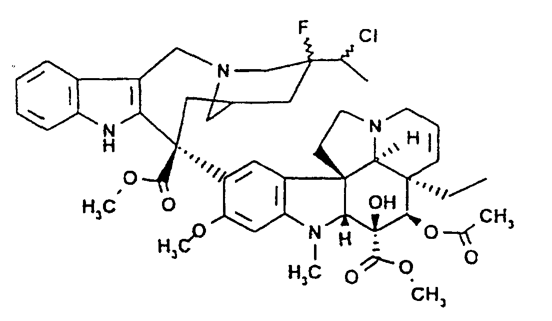

3. Procédé de préparation des composés selon les revendications 1 et 2, **caractérisé en ce que** l'on fait réagir un composé de formule générale 2 en milieu superacide, provenant de l'association d'un acide de Bronsted comme l'acide fluorhydrique HF et d'un acide de Lewis comme le pentafluorure d'antimoine SbF₅, en présence d'un dérivé chloré générateur d'ions superélectrophiles selon le schéma : 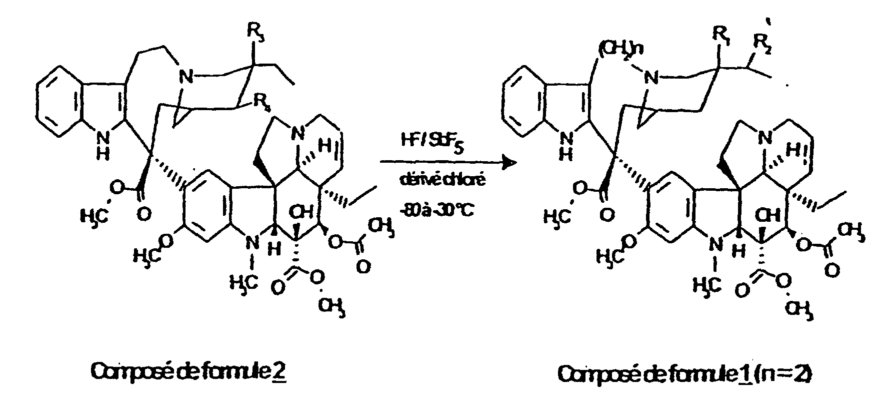 où R₃ représente un groupement hydroxyle et R₄ un atome d'hydrogène ou bien R₃ et R₄ forment une double liaison et où R₁ et R₂ sont tels que définis à la revendication 1.

4. Procédé de préparation de composés selon la revendication 3, **caractérisé en ce que** la réaction d'un composé de formule générale **2** en milieu superacide s'effectue à une température comprise entre -80 et -30 °C.

5. Procédé de préparation de composés selon la revendication 3, **caractérisé en ce que** le dérivé chloré générateur d'ions superélectrophiles est choisi parmi :
- le chlorure de méthylène,
- le chloroforme,
- le tétrachlorure de carbone,
- le 2,2-dichloropropane,
ou un mélange de ces dérivés en toutes proportions.

6. Procédé de préparation de composés selon les revendications 3 et 5, **caractérisé en ce que** le dérivé chloré générateur d'ions superétectrophiles utilisé pour obtenir les composés de formule générale **1** dans laquelle **n** = 2 est de préférence le chlorure de méthylène, le tétrachlorure de carbone, ou un mélange de ces dérivés en toutes proportions.

7. Procédé de préparation de composés selon la revendication 1, **caractérisé en ce que** l'on fait réagir un composé de formule générale **1** dans laquelle **n** = 2, **R**_{**1**} et **R**_{**2**} étant défini comme ci-dessus, avec agent halogénant comme la N-bromosuccinimide, dans un solvant chloré comme le chlorure de méthylène et en présence d'un acide comme l'acide trifluoroacétique à une température inférieure à -10 °C, conduisant à la 9'-bromoindolénine correspondante, intermédiaire non isolé.

8. Procédé de préparation de composés selon la revendication 1 et 7, **caractérisé en ce que** l'on neutralise le mélange réactionnel contenant la 9'-bromoindolénine du composé de formule générale **1** dans laquelle **n** = 2 par un milieu basique, suivi d'une hydrolyse de préférence par un mélange [chlorure de méthylène : eau : tétrahydrofuranne], en présence ou non de tétrafluoroborate d'argent à une température comprise entre -10 °C et le reflux du solvant, pour obtenir le composé de formule générale **1** dans laquelle **n** = 1, selon le schéma suivant :

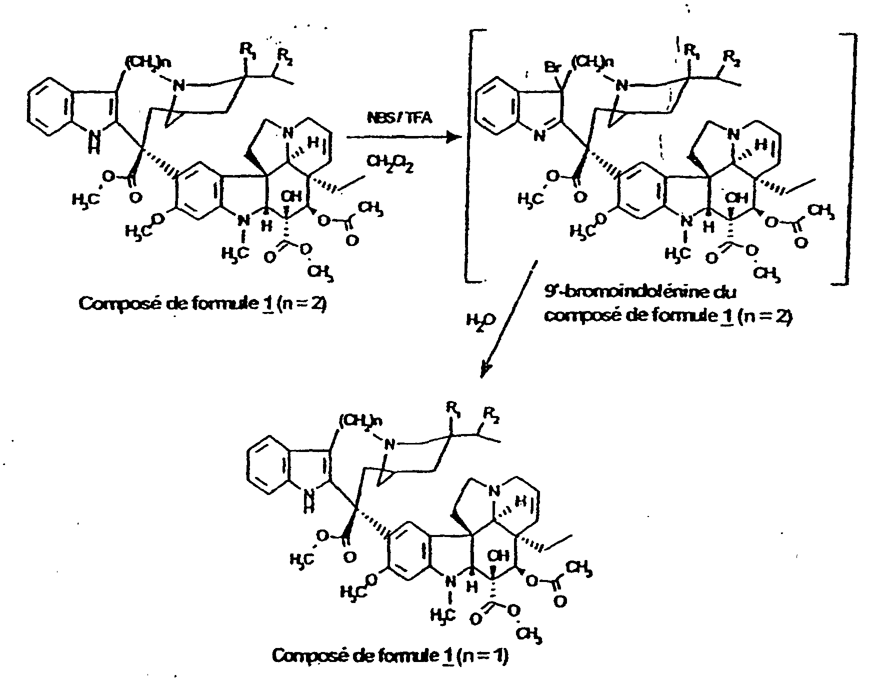

9. A titre de médicaments nouveaux utiles, par exemple en thérapeutique humaine dans le traitement de la pathologie cancéreuse, les composés définis selon l'une des revendications 1 et 2.

10. Composition pharmaceutique **caractérisée en ce qu'**elle contient à titre de principe actif au moins un composé selon l'une des revendications 1 et 2, associé à un véhicule pharmaceutique acceptable.

## Patentansprüche

1. Antimitotische Derivate von Vinca-Alkaloiden, welche der folgenden allgemeinen Formel entsprechen: 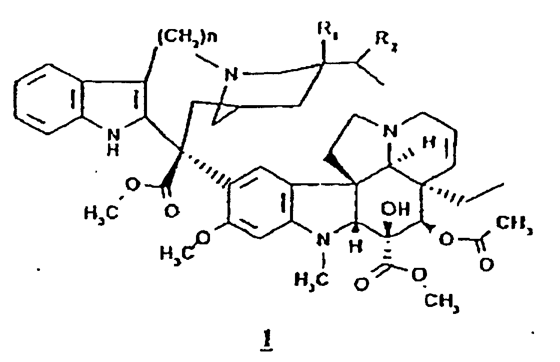 in welcher:
n = 1 oder 2,
R₁ für ein Wasserstoff- oder Fluoratom steht,
R₂ für ein Chloratom steht,
sowie deren Salze mit therapeutisch annehmbaren anorganischen oder organischen Säuren und die Mischungen der Diastereomeren, welche den Konfigurationen der 4'- und 20'-Kohlenstoffatome der Verbindungen entsprechen, und deren Mischungen in einem jeglichen Verhältnis.

2. Verbindungen der allgemeinen Formel **1** nach Anspruch 1, **dadurch gekennzeichnet, dass** sie ausgewählt werden unter:
20'-Chlor-4'-desoxy-4'-fluorvinblastin: 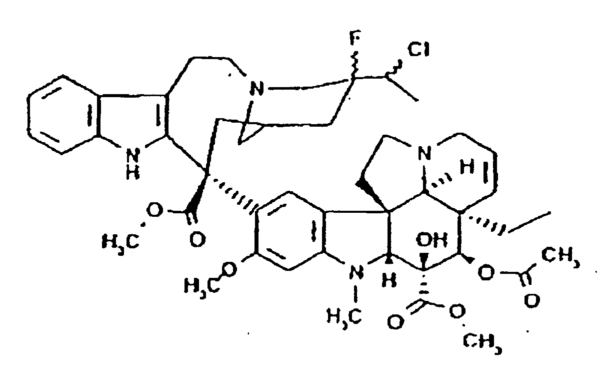 20'-Chlor-4'-desoxyvinblastin: 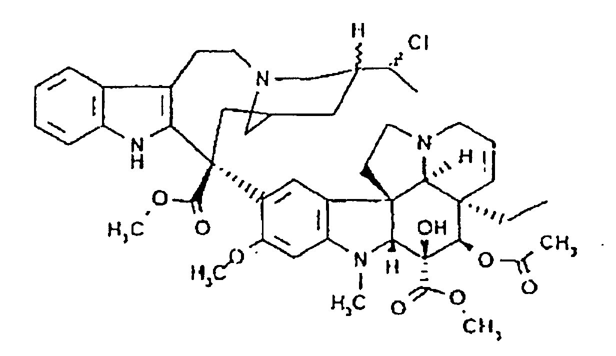 20'-Chlor-3',4'-dihydrovinorelbin: 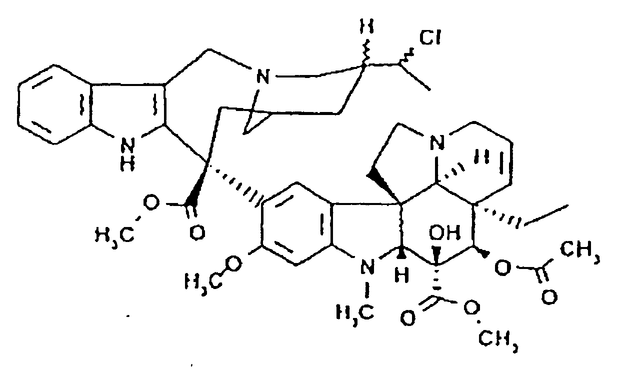 20'-Chlor-3',4'-dihydro-4'-fluorvinorelbin:

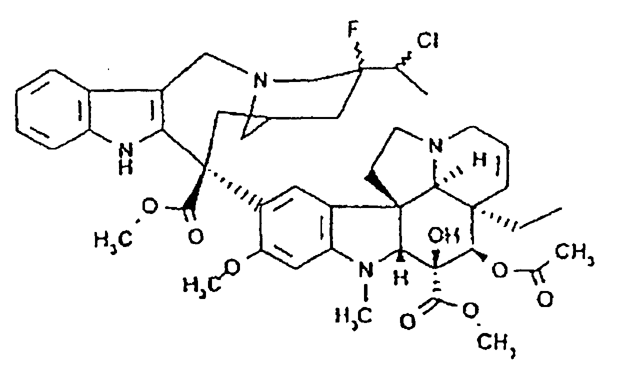

3. Verfahren zur Herstellung der Verbindungen nach den Ansprüchen 1 und 2, **dadurch gekennzeichnet, dass** man eine Verbindung der allgemeinen Formel **2** in Supersäure-Milieu, welches aus der Kombination einer Brönsted-Säure, wie Fluorwasserstoffsäure, HF, und einer Lewis-Säure, wie Antimonpentafluorid, SbF₅, hervorgeht, in Gegenwart eines chlorhaltigen Derivats, welches superelektrophile Ionen erzeugt, reagieren lässt gemäß dem Schema: 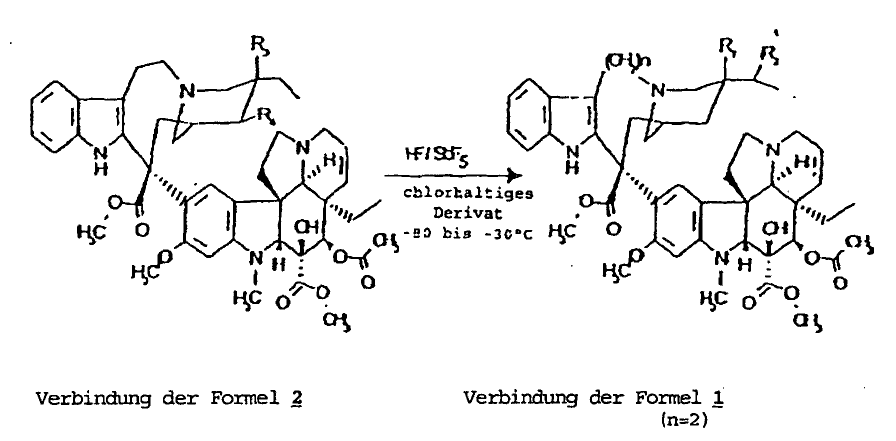 worin R₃ für eine Hydroxylgruppe und R₄ für ein Wasserstoffatom steht oder alternativ R₃ und R₄ eine Doppelbindung bilden und wobei R₁ und R₂ wie in Anspruch 1 definiert sind.

4. Verfahren zur Herstellung von Verbindungen nach Anspruch 3, **dadurch gekennzeichnet, dass** die Umsetzung einer Verbindung der allgemeinen Formel **2** in Supersäure-Milieu bei einer Temperatur zwischen -80 und -30°C erfolgt.

5. Verfahren zur Herstellung von Verbindungen nach Anspruch 3, **dadurch gekennzeichnet, dass** das chlorhaltige Derivat, welches superelektrophile Ionen erzeugt, ausgewählt wird unter:
- Methylenchlorid,
- Chloroform,
- Tetrachlorkohlenstoff,
- 2,2-Dichlorpropan
oder Mischungen von diesen Derivaten in jeglichen Verhältnissen.

6. Verfahren zur Herstellung von Verbindungen nach den Ansprüchen 3 und 5, **dadurch gekennzeichnet, dass** das superelektrophile Ionen erzeugende chlorhaltige Derivat, welches eingesetzt wird, um die Verbindungen der allgemeinen Formel **1**, in welcher n = 2, zu erhalten, vorzugsweise Methylenchlorid, Tetrachlorkohlenstoff oder eine Mischung dieser Derivate in jeglichen Verhältnissen ist.

7. Verfahren zur Herstellung von Verbindungen nach Anspruch 1, **dadurch gekennzeichnet, dass** man eine Verbindung der allgemeinen Formel **1**, in welcher n = 2, wobei R₁ und R₂ wie oben definiert sind, mit einem Halogenierungsmittel, wie N-Bromsuccinimid, in einem chlorierten Lösemittel, wie Methylenchlorid, und in Gegenwart einer Säure, wie Trifluoressigsäure, bei einer Temperatur unter -10°C reagieren lässt, was zu dem entsprechenden 9'-Bromindolenin, einem Zwischenprodukt, das nicht isoliert wird, führt.

8. Verfahren zur Herstellung von Verbindungen nach Anspruch 1 und 7, **dadurch gekennzeichnet, dass** man die das 9'-Bromindolenin der Verbindung der allgemeinen Formel **1**, in welcher n = 2, enthaltende Reaktionsmischung durch ein basisches Medium neutralisiert, gefolgt von einer Hydrolyse vorzugsweise durch eine Mischung aus [Methylenchlorid : Wasser : Tetrahydrofuran] in Gegenwart von Silbertetrafluorborat oder nicht bei einer Temperatur zwischen - 10°C und der Rückflusstemperatur des Lösemittels, um die Verbindung der allgemeinen Formel **1**, in welcher n = 1, gemäß dem folgenden Schema zu erhalten:

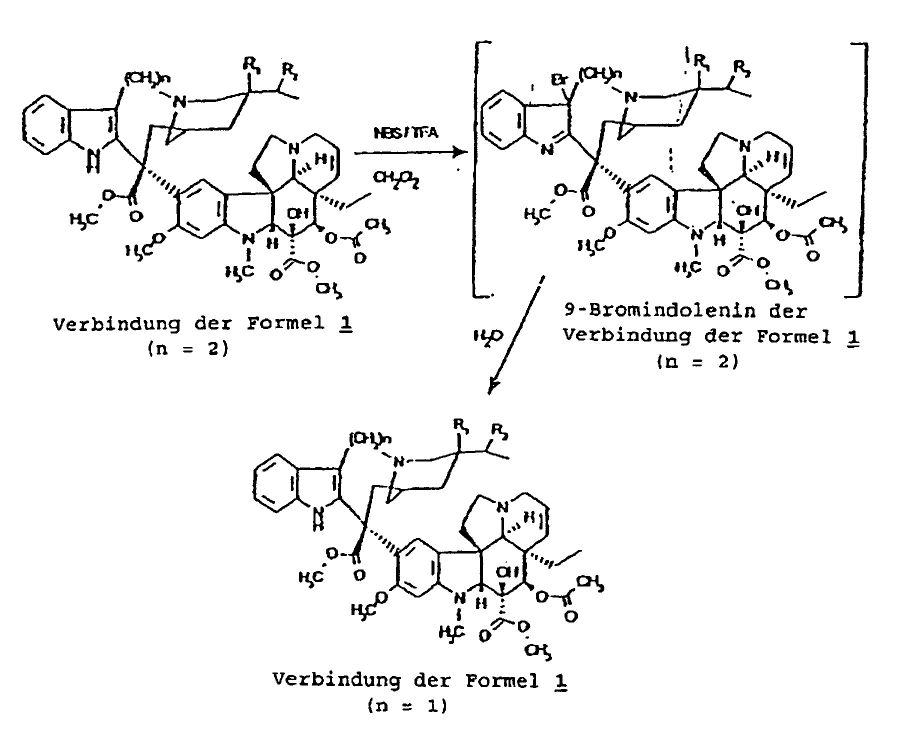

9. Gemäß einem der Ansprüche 1 und 2 definierte Verbindungen als neue Arzneimittel, welche beispielsweise bei der Therapie von Menschen bei der Behandlung von Krebserkrankungen nützlich sind.

10. Pharmazeutische Zusammensetzung, **dadurch gekennzeichnet, dass** sie als Wirkstoff wenigstens eine Verbindung nach einem der Ansprüche 1 und 2 in Kombination mit einem pharmazeutisch annehmbaren Träger enthält.

## Claims

1. Antimitotic derivatives of Vinca alkaloids corresponding to the general formula below: 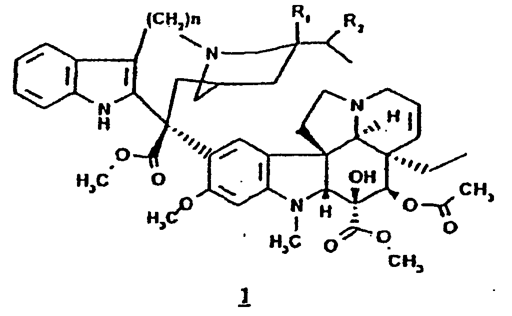 in which:
n = 1 or 2,
R₁ represents a hydrogen or fluorine atom,
R₂ represents a chlorine atom,
as well as the salts thereof with therapeutically acceptable inorganic or organic acids, and the mixtures of the diastereoisomers corresponding to the configurations of the 4' and 20' carbons of the compounds and their mixtures in any proportion.

2. Compounds of general formula 1 according to Claim 1, **characterized in that** they are chosen from: 20'-chloro-4'-deoxy-4'-fluorovinblastine, 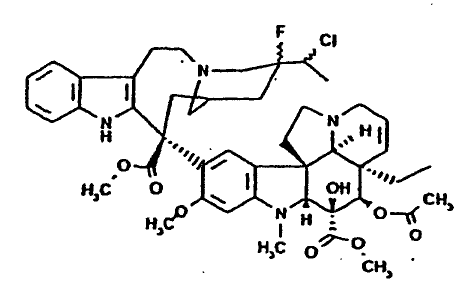 20'-chloro-4'-deoxyvinblastine: 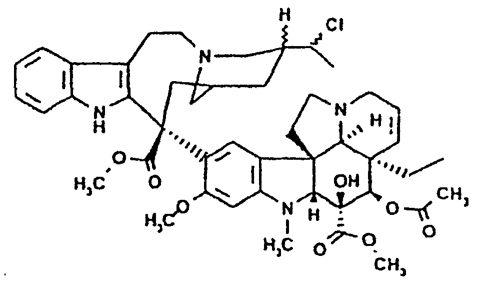 20'-chloro-3',4'-dihydrovinorelbine: 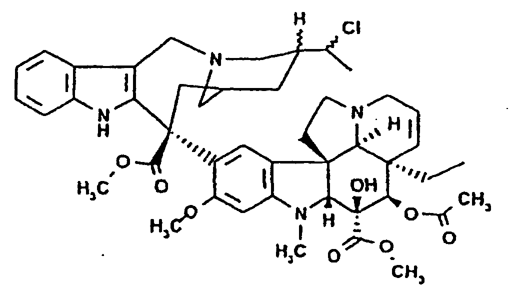 20'-chloro-3',4'-dihydro-4'-fluorovinorelbine:

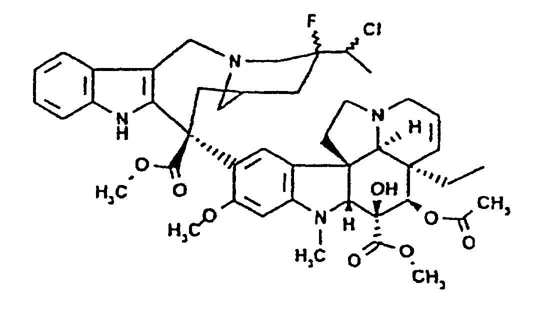

3. Process for preparing the compounds according to Claims 1 and 2, **characterized in that** a compound of general formula 2 is reacted in superacid medium, obtained by combining a Bronsted acid such as hydrofluoric acid HF, and a Lewis acid such as antimony pentafluoride SbF₅, in the presence of a chloro derivative which generates superelectrophilic ions, according to the scheme: 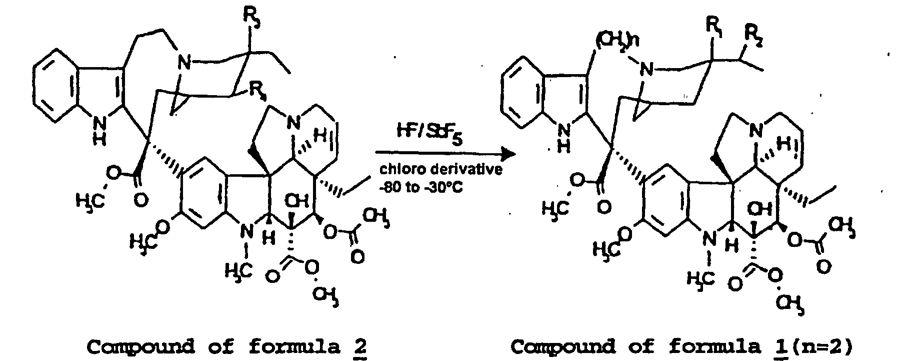 where R₃ represents a hydroxyl group and R₄ a hydrogen atom, or else R₃ and R₄ form a double bond and where R₁ and R₂ are as defined in Claim 1.

4. Process for preparing the compounds according to Claim 3, **characterized in that** the reaction of a compound of general formula 2 in superacid medium is carried out at a temperature of between -80 and -30°C.

5. Process for preparing the compounds according to Claim 3, **characterized in that** the chloro derivative which generates superelectrophilic ions is chosen from:
- methylene chloride,
- chloroform,
- carbon tetrachloride,
- 2,2-dichloropropane, or a mixture of these derivatives in all proportions.

6. Process for preparing the compounds according to Claims 3 and 5, **characterized in that** the chloro derivative which generates superelectrophilic ions, which is used to obtain the compounds of general formula 1 in which n = 2, is preferably methylene chloride, carbon tetrachloride or a mixture of these derivatives in all proportions.

7. Process for preparing the compounds according to Claim 1, **characterized in that** a compound of general formula 1 in which n = 2, R₁ and R₂ being defined as above, is reacted with a halogenating agent such as N-bromosuccinimide, in a chlorinated solvent such as methylene chloride and in the presence of an acid such as trifluoroacetic acid at a temperature below -10°C, leading to the corresponding 9'-bromoindolenine, which intermediate is not isolated.

8. Process for preparing the compounds according to Claims 1 and 7, **characterized in that** the reaction mixture containing the 9'-bromoindolenine of the compound of general formula 1 in which n = 2 is neutralized with a basic medium, followed by a hydrolysis preferably with a mixture [methylene chloride:water:tetrahydrofuran], in the presence or absence of silver tetrafluoroborate at a temperature of between -10°C and the reflux point of the solvent, in order to obtain the compound of general formula 1 in which n = 1, according to the scheme below:

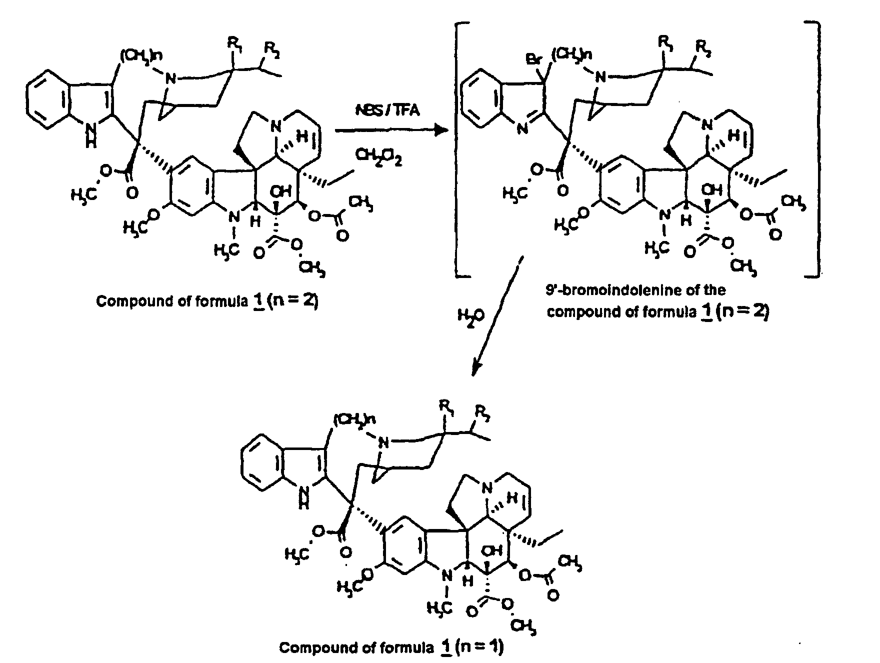

9. As novel medicinal products which are useful, for example, in human therapy in the treatment of cancer pathology, the compounds defined in either of Claims 1 and 2.

10. Pharmaceutical composition **characterized in that** it contains, as active principle, at least one compound according to either of Claims 1 and 2, combined with a pharmaceutically acceptable vehicle.
